(19) Europäisches Patentamt / European Patent Office / Office européen des brevets

(11) **EP 2 475 978 B1**

(12) **EUROPÄISCHE PATENTSCHRIFT**

(45) Veröffentlichungstag und Bekanntmachung des Hinweises auf die Patenterteilung:
**19.02.2020   Patentblatt 2020/08**

(21) Anmeldenummer: **10749751.3**

(22) Anmeldetag: **23.08.2010**

(51) Int Cl.:
**G01N 21/89** *(2006.01)*     **G01N 33/36** *(2006.01)*

(86) Internationale Anmeldenummer:
**PCT/CH2010/000205**

(87) Internationale Veröffentlichungsnummer:
**WO 2011/026249 (10.03.2011 Gazette 2011/10)**

(54) **VORRICHTUNG UND VERFAHREN ZUR OPTISCHEN ABTASTUNG EINES BEWEGTEN TEXTILMATERIALS**

DEVICE AND METHOD FOR OPTICALLY SCANNING A MOVING TEXTILE MATERIAL

DISPOSITIF ET PROCÉDÉ POUR RÉALISER LE BALAYAGE OPTIQUE D'UN TEXTILE EN MOUVEMENT

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **07.09.2009   CH 13882009**

(43) Veröffentlichungstag der Anmeldung:
**18.07.2012   Patentblatt 2012/29**

(73) Patentinhaber: **Uster Technologies AG**
**8610 Uster (CH)**

(72) Erfinder:
- **PIRANI, Peter**
  **CH-8624 Grüt (CH)**
- **WAMPFLER, Hansruedi**
  **CH-8049 Zürich (CH)**

(56) Entgegenhaltungen:
**EP-A1- 0 652 432     US-A1- 2006 164 646**

**Beschreibung**

FACHGEBIET

Die vorliegende Erfindung liegt auf dem Gebiet der textilen Materialprüfung. Sie betrifft eine Vorrichtung und ein Verfahren zur optischen Abtastung eines bewegten Textilmaterials, gemäss den Oberbegriffen der unabhängigen Patentansprüche. Die Erfindung kann bspw. in optischen Garnreinigern auf Spinn- oder Spulmaschinen eingesetzt werden.

STAND DER TECHNIK

Es ist eine Vielzahl verschiedenartiger Vorrichtungen und Verfahren zur Prüfung von Textilmaterialien bekannt. Sie lassen sich nach ihrer Anwendung in die beiden Klassen Laborprüfung (offline) und Prüfung während des Produktionsprozesses (online) einteilen. In den Textilprüfvorrichtungen kommen verschiedene Sensorprinzipien zur Anwendung; der Einsatz eines bestimmten Sensorprinzips hängt unter anderem davon ab, welche Eigenschaft optimal detektiert werden soll. Häufig verwendete Sensorprinzipien, besonders in der Gamprüfung, sind das kapazitive und das optische. Beim letzteren wird das Garn von einer Lichtquelle beleuchtet, und mit dem Garn wechselwirkendes Licht wird von einem Lichtdetektor detektiert. Daraus lässt sich bspw. die Garndicke oder das Vorhandensein von Fremdstoffen bestimmen.

Die WO-93/13407 A1 gibt ein Beispiel für einen optischen Garnreiniger zur Detektion von Fremdfasern an. Das durch einen Messspalt bewegte Garn wird von einer Lichtquelle mit moduliertem Licht beleuchtet. Ein erster Sensor nimmt vom Garn reflektiertes Licht und gleichzeitig ein zweiter Sensor vom Garn transmittiertes Licht auf. Die von den beiden Sensoren ausgegebenen elektrischen Signale werden so miteinander zu einem Ausgangssignal verknüpft, z. B. addiert, dass das Ausgangssignal die Anwesenheit einer Fremdfaser im Garn anzeigt, von Durchmesseränderungen des Garns jedoch unabhängig ist. Es sind weitere optische Garnreiniger zur Detektion von Verunreinigungen bekannt, die auf demselben Messprinzip beruhen. Dabei werden jeweils zwei verschiedene optische Signale von Licht, das mit dem Garn wechselwirkt, detektiert und so miteinander verknüpft, dass das Verknüpfungssignal nur eventuelle Verunreinigungen, nicht aber Durchmesseränderungen anzeigt. Gemäss der US-5,383,017 A und der US-5,414,520 A ist die Verknüpfung eine Quotientenbildung. Die US-5,768,938 A sieht ausserdem Lichtübertragungselemente zur Lichtführung zwischen Messspalt und Lichtquelle bzw. Sensoren vor, welche Lichtübertragungselemente in einer Ebene angeordnet sind, die senkrecht zum Garn steht.

Die optischen Messköpfe der Garnreiniger gemäss der EP-1'655'599 A2 und der EP-1'655'600 A2 sind demjenigen der US-5,768,938 A nachempfunden. In beiden wird das Garn von einer Lichtquelle beleuchtet. Ein erster, der Lichtquelle gegenüber liegender Lichtempfänger detektiert vom Garn transmittiertes Licht, zwei weitere, neben der Lichtquelle angeordnete Lichtempfänger detektieren vom Garn reflektiertes Licht. Lichtübertragungselemente dienen der Lichtführung zwischen Messspalt und Lichtquelle bzw. Lichtempfängern. Gemäss der EP-1'655'599 A2 ist der Messkopf so ausgebildet, dass durch die Lichtempfänger in Abwesenheit des Garnes jeweils Abbildungen der gegenüberliegenden Wand des Messspaltes erfassbar sind, die im Wesentlichen beidseitig ausserhalb der von der direkten Strahlung der Lichtquelle beleuchteten Fläche der Wand des Messspaltes liegen. Dadurch wird der Störeinfluss parasitärer Signale unterdrückt und die Fremdfasererkennung verbessert. Die EP-1'655'600 A2 schlägt vor, die Lichtquelle als Strahler mit Lambertscher Abstrahlcharakteristik auszubilden. Dasjenige Lichtübertragungselement, welches Licht vom Messspalt zum ersten Lichtempfänger führt, enthält eine Blende und eine Linse, wobei die Blende im Unendlichen abgebildet wird. Zwischen der Lichtquelle und der Blende ist ein Diffusor angeordnet, der eine die Blende passierende Strahlung erzeugt, die symmetrisch zur optischen Achse der Linse ist. So wird die Qualität der Messergebnisse verbessert.

Die WO-00/20849 A1 offenbart eine Vorrichtung zum Erfassen von Fremdstoffen in einem Garn. Ein Beleuchtungselement ist halbkugelförmig über dem Garn ausgebildet und weist über die Halbkugel verteilte Lichtquellen derselben Lichtwellenlänge auf. Alle Lichtquellen sind auf das Zentrum der Halbkugel gerichtet, durch welches das Garn bewegt wird. Ein Objektiv bildet den beleuchteten Garnabschnitt auf einen Detektor ab. In der Umgebung des Zentrums entsteht ein besonders heller und homogener Lichtfleck, der zwecks besserer Ortsauflösung durch vor dem Detektor angebrachte Blenden beschränkt werden kann. Diese Vorrichtung ist kompakt und Platz sparend. Sie erreicht eine intensive Beleuchtung des abgebildeten Gamabschnitts, so dass das Garn mit hoher Geschwindigkeit bewegt werden kann und Fremdstoffe trotzdem zuverlässig erfasst werden.

Ferner sind viele optische Garnreiniger zur Detektion von Fremdstoffen im Garn bekannt, welche mit zwei oder mehr verschiedenen Lichtwellenlängen arbeiten, um die Fremdstoffe zuverlässiger vom Grundmaterial des Garns und/oder voneinander zu unterscheiden. Die Garnreiniger gemäss der EP-0'399'945 A2 oder der EP-0'652'432 A1 verwenden jeweils eine breitbandige Lichtquelle und zwei wellenlängenselektive Lichtdetektoren, von denen der eine z. B. für rotes und der andere für grünes Licht empfindlich ist. Gleiche Änderungen der beiden Detektorsignale deuten bloss auf eine Änderung des Garndurchmessers hin, unterschiedliche Änderungen jedoch auf Fremdstoffe im Garn. Die physikalische Umkehr dieser Anordnung ergibt eine zweite Ausführungsform, in welcher eine rote und eine grüne Lichtquelle, die nacheinander gepulst betrieben werden, und ein breitbandiger Lichtdetektor vorhanden sind. Die WO-00/73771 A1 verwendet zwei Wellenlängen im Infrarotbereich (IR), um Polypropylen in Baumwolle besser detektieren zu können. Gemäss der WO-2007/010325 A1 liegt die eine Lichtkomponente im sichtbaren, die zweite im infraroten (IR) oder im ultravioletten (UV) Bereich des elektromagnetischen Spektrums. Die WO-03/008950 A2 schlägt vor, die beiden Lichtkomponenten nicht separat, sondern gleichzeitig und gemeinsam zu erfassen, wobei verschiedene Fremdstoffe aufgrund unterschiedlicher Signalausschläge voneinander unterschieden werden können. Die Fremdstoffdetektion mit zwei Lichtwellenlängen kann auf drei, z. B. die Farben Rot, Grün und Blau, erweitert werden. Damit kann bereits die Farbe des Garns annähernd bestimmt werden. Beispiele dafür geben die EP-1'018'645 A1, die WO-95/29396 A1, die WO-2004/044579 A1 und die WO-2007/012936 A2 an. Bei den oben beschriebenen Fremdstoffreinigern wird das Garn mit Licht, welches zwei oder mehr Komponenten mit verschiedenen Wellenlängen oder Farben beinhaltet, beleuchtet. Zur Erzeugung dieses Lichtes kommen entweder mehrere schmalbandige, nacheinander betriebene Lichtquellen (z. B. Leuchtdioden, abgekürzt LEDs) oder eine breitbandige Lichtquelle (z. B. Glühlampe, Multi-LED oder Lumineszenzkonversions-LED) in Frage.

Die Wellenlängenselektion erfolgt im ersteren Fall zeitlich, durch den sequenziellen Betrieb der Lichtquellen, im letzteren Fall örtlich, durch wellenlängenselektive Lichtdetektoren.

Die WO-2007/012936 A2 lehrt die Verwendung einer RGB-Leuchtdiode, bei welcher drei verschiedenfarbige lichtemittierende Dioden an verschiedenen Orten in einem gemeinsamen transparenten Gehäuse untergebracht sind.

Gemäss der EP-0'652'432 A1 wird ein ausgeleuchteter Bereich eines Garns von einer polychromaten Lichtquelle durch eine Lichtaustrittsöffnung beleuchtet. Eine Sensoreinrichtung mit vertikal gestaffelt angeordneten wellenlängenselektiven Photodioden tastet durch eine Lichteintrittsöffnung einen erfassbaren Bereich des Garns ab. Der ausgeleuchtete Bereich und der erfassbare Bereich des Garns sind identisch und bilden einen gemeinsamen Messbereich. Dadurch werden Einflüsse, die durch die Garnstruktur bedingt sind, weitestgehend ausgeschaltet.

Im Fremdstoffreiniger mit mehreren Lichtquellen sind die verschiedenen Lichtquellen zwangsläufig an verschiedenen, wenn auch nahe beieinander liegenden Orten angeordnet. Falls das Licht dieser Lichtquellen von einer gemeinsamen Linse kollimiert wird, so ergeben sich hinter der Linse verschiedenfarbige Teilstrahlen, die mit unterschiedlichen Abstrahlungswinkeln von der Linse zum Garn hin verlaufen. Demzufolge gibt es auf dem im Messfeld Bereiche, die nur von einem dieser Teilstrahlen, von mehreren davon bzw. von allen Teilstrahlen beleuchtet werden. Deshalb kann es geschehen, dass sich ein im Garn vorhandener Fremdstoff während seiner Erfassung durch den Fremdstoffreiniger in einem Bereich befindet, der nicht von allen Teilstrahlen beleuchtet wird. Dies kann zu falschen Messresultaten führen und ist unerwünscht. Im Idealfall sollte sich der Fremdstoff während seiner Erfassung immer in demjenigen Bereich befinden, der von allen Teilstrahlen beleuchtet wird, was jedoch beim Stand der Technik nicht gewährleistet ist. Das Problem kann einerseits in Längsrichtung des Garns auftreten, weil ja das Garn in der Längsrichtung durch das Messfeld hindurch bewegt wird. Es kann aber andererseits auch in Richtung quer zur Längsrichtung auftreten, weil der Garnlauf in Querrichtung selten stabil ist.

Man könnte dem Problem auszuweichen versuchen, indem man die verschiedenfarbigen Bereiche im Messfeld sogar absichtlich lokal voneinander trennt und den Sampleversatz der zeitlich nacheinander erfolgenden Messungen in den verschiedenfarbigen Bereichen durch eine rechnerische Korrektur zur zeitlichen Übereinstimmung bringt. Für eine solche Korrektur des Sampleversatzes muss man jedoch die momentane Garngeschwindigkeit kennen, was selten der Fall ist. Eine Durchschnittsgeschwindigkeit des Garns genügt hierzu nicht, weil die Momentangeschwindigkeit aufgrund der seitlichen Verlegung des Garns auf eine Spule um bis zu $\pm 30$ % von der Durchschnittsgeschwindigkeit abweichen kann.

DARSTELLUNG DER ERFINDUNG

Es ist eine Aufgabe der vorliegenden Erfindung, eine Vorrichtung und ein Verfahren zur optischen Abtastung eines bewegten Textilmaterials anzugeben, welche gewährleisten, dass das Textilmaterial mit allen beteiligten Lichtkomponenten abgetastet wird. Die den verschiedenen Lichtkomponenten zugeordneten Sensorsignale sollen einfach und genau miteinander verknüpft werden können, ohne die Momentangeschwindigkeit des Textilmaterials genau zu kennen. Die Vorrichtung und das Verfahren sollen gegen Garnlaufschwankungen robust sein.

Diese und andere Aufgaben werden durch die erfindungsgemässe Vorrichtung und das erfindungsgemässe Verfahren, wie sie in den unabhängigen Patentansprüchen definiert sind, gelöst. Vorteilhafte Ausführungsformen sind in den abhängigen Patentansprüchen angegeben.

Gemäss der Erfindung wird durch mindestens eine Feldblende nur derjenige Bereich des beleuchteten Messfeldes ausgewählt, der von allen Lichtquellen beleuchtbar ist. Dies hat zur Folge, dass das ganze Sichtfeld des Detektors mit allen beteiligten Farben ausgeleuchtet ist. Der Detektor sieht keine Stelle, die z. B. nur von einer Farbe beleuchtet ist. Eine Korrektur des Sampleversatzes erübrigt sich. Es ist, als wären die mindestens zwei Lichtquellen am selben Ort angeordnet. Die erfindungsgemässe Massnahme kann für alle in der Vorrichtung eingesetzten Detektoren eingesetzt werden, sowohl für Durchlicht- als auch für Auflichtdetektoren.

Unter einer Feldblende wird in der technischen Optik bekanntlich ein die Ausdehnung des Strahlenbündels begrenzendes Element verstanden, welches die Ausdehnung desjenigen Bereichs des Objektes bestimmt, der abgebildet werden kann. Üblicherweise schattet eine Feldblende Randbereiche des Abbildes ab. Man unterscheidet zwischen Gesichtsfeldblenden und Bildfeldblenden. Eine Gesichtsfeldblende ist die Eintrittsöffnung des Systems und begrenzt den maximalen Bildwinkel, den das System abbildet. Eine Bildfeldblende ist die Austrittsöffnung des Systems und begrenzt ebenfalls den maximalen Bildwinkel, nachdem die Strahlen durch das System getreten sind. Eine Aperturblende bestimmt hingegen die Lichtmenge im Bild. Die Begriffe "Feldblende" und "Aperturblende", wie sie in der vorliegenden Schrift verwendet werden, sind z. B. detailliert erklärt in E. Hecht, A. Zajac, "Optics", Addison-Wesley Publishing Company, 1974, Absatz 5.3.

Dementsprechend beinhaltet die erfindungsgemässe Vorrichtung zur optischen Abtastung eines bewegten Textilmaterials einen Pfad für das Textilmaterial, mindestens zwei Lichtquellen, die an verschiedenen Orten angeordnet sind und Licht mit verschiedenen spektralen Eigenschaften aussenden, zur Beleuchtung jeweils eines Beleuchtungsbereichs des Pfades, und einen Lichtdetektor zur Detektion von aus den verschiedenen Beleuchtungsbereichen stammendem Licht. Mindestens eine Feldblende lässt nur Licht aus einem Abtastbereich, der eine Teilmenge der Schnittmenge aller Beleuchtungsbereiche ist, auf den Lichtdetektor auftreffen. Bei der Teilmenge kann es sich um eine echte Teilmenge oder um eine mit der Schnittmenge identische Menge handeln.

Die mindestens eine Feldblende kann durch eine Gesichtsfeldblende und/oder eine Bildfeldblende realisiert sein.

Die Lichtquellen, der Pfad und der Lichtdetektor können derart gegenseitig angeordnet sein, dass der Lichtdetektor am Textilmaterial transmittiertes oder vom Textilmaterial reflektiertes Licht detektiert. In einer bevorzugten Ausführungsform sind mehrere Lichtdetektoren vorhanden, von denen mindestens einer, vorzugsweise genau einer, am Textilmaterial transmittiertes und mindestens ein anderer, vorzugsweise genau zwei, vom Textilmaterial reflektiertes Licht detektieren. Zur Lichtführung zwischen dem Pfad für das Textilmaterial und den Lichtquellen bzw. dem Lichtdetektor können Lichtübertragungselemente, bspw. Lichtleiter aus Kunststoff, vorhanden sein.

Es ist vorteilhaft, wenn jede der Lichtquellen Licht aussendet, dessen Wellenlängenspektrum im Wesentlichen ein zusammenhängender, begrenzter Spektralbereich ist, wobei sich die Spektralbereiche der verschiedenen Lichtquellen im Wesentlichen nicht überlappen. Dementsprechend sind die Lichtquellen vorzugsweise als Leuchtdioden (LEDs) ausgebildet. In einer bevorzugten Ausführungsform ist eine rotes Licht aussendende und eine grünes Licht aussendende Leuchtdiode vorhanden. Die Lichtquellen können in einem gemeinsamen Gehäuse untergebracht sein, das vorzugsweise voll vergossen und für das ausgesendete Licht transparent ist. Das Gehäuse kann gleichzeitig als Kollimatorlinse für das ausgesendete Licht wirken.

Die erfindungsgemässe Vorrichtung wird z. B. zur Detektion von Fremdstoffen im Textilmaterial, vorzugsweise in Garn, verwendet. Sie kommt mit Vorteil in Garnreinigern auf Spinn- oder Spulmaschinen zum Einsatz.

Im erfindungsgemässen Verfahren zur optischen Abtastung eines bewegten Textilmaterials wird das Textilmaterial von mindestens zwei Lichtkomponenten mit verschiedenen spektralen Eigenschaften aus mindestens zwei Lichtquellen, die an verschiedenen Orten angeordnet sind, in jeweils einem Beleuchtungsbereich beleuchtet. Aus den verschiedenen Beleuchtungsbereichen stammendes Licht wird detektiert, und zwar nur Licht aus einem Abtastbereich, der eine Teilmenge der Schnittmenge aller Beleuchtungsbereiche ist. Das detektierte Licht kann dazu verwendet werden, Fremdstoffe im Textilmaterial, das vorzugsweise ein Garn ist, zu detektieren.

Im vorliegenden Dokument werden Begriffe wie "Licht" oder "beleuchten" nicht nur für sichtbares Licht, sondern auch für elektromagnetische Strahlung aus den angrenzenden Spektralbereichen Ultraviolett (UV) und Infrarot (IR) verwendet.

## AUFZÄHLUNG DER ZEICHNUNGEN

Nachfolgend wird die Erfindung anhand der schematischen Zeichnungen detailliert erläutert.

Figur 1     zeigt eine erste Ausführungsform der erfindungsgemässen Vorrichtung mit einer Transmissionsabtastung in einer Seitenansicht.

Figur 2     zeigt die verschiedenen Beleuchtungsbereiche der Vorrichtung von Figur 1 in einer Frontalansicht.

Figur 3     zeigt eine zweite Ausführungsform der erfindungsgemässen Vorrichtung mit einer Reflexionsabtastung in einer Seitenansicht.

Figur 4     zeigt eine dritte Ausführungsform der erfindungsgemässen Vorrichtung mit einer Transmissionsabtastung in einer Seitenansicht.

Figur 5     zeigt den detektorseitigen Teil einer vierten Ausführungsform der erfindungsgemässen Vorrichtung mit einer Transmissionsabtastung in einer Seitenansicht.

## AUSFÜHRUNG DER ERFINDUNG

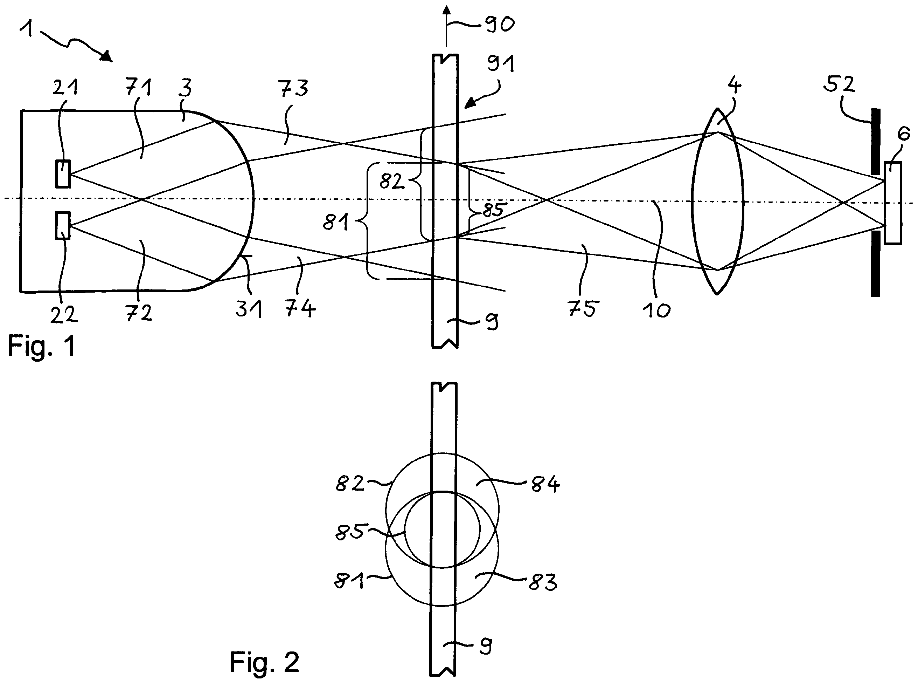

In **Figur 1** ist eine erste Ausführungsform der erfindungsgemässen Vorrichtung 1 in einer Seitenansicht dargestellt. Ein Textilmaterial 9, z. B. ein Garn, wird entlang eines vorbestimmten Pfades 91 durch die Vorrichtung 1 bewegt. In Figur 1 ist die Bewegungsrichtung, die identisch ist mit der Längsrichtung des Garns 9, mit einem Pfeil 90 angedeutet.

Zur Beleuchtung des Garns 9 bzw. des Pfades 91 beinhaltet die Vorrichtung 1 zwei verschiedenfarbige Lichtquellen 21, 22. Im vorliegenden Ausführungsbeispiel handelt es sich um zwei Leuchtdioden (LEDs), bspw. eine erste LED 21, die rotes Licht 71 aussendet, und eine zweite LED 22, die grünes Licht 72 aussendet. Die Lichtquellen 21, 22 sind vorzugsweise mittels einer (nicht eingezeichneten) Lichtquellensteuerung einzeln ansteuerbar. Vorzugsweise wird ihre Intensität periodisch moduliert. Bei zwei Lichtquellen 21, 22 können die Intensitätsverläufe um 90° phasenverschoben sein, so dass eine Quadratur-Demodulation vorgenommen werden kann. Alternativ können die Lichtquellen 21, 22 nacheinander ein- und ausgeschaltet werden. Wichtig ist es, die Frequenz der periodischen Lichtquellenmodulation derart hoch zu wählen, dass das Textilmaterial 9 innerhalb einer Periode nur eine Strecke zurücklegt, die klein ist im Vergleich zur Ausdehnung eines Abtastbereiches 85, der von beiden Lichtquellen 21, 22 beleuchtet wird, vorzugsweise mindestens zehnmal kleiner. Betrachten wir ein Beispiel, in dem sich das Garn 9 mit einer Geschwindigkeit von v = 2000 m/min = 33 m/s durch die Vorrichtung 1 bewegt und mit einer Abtastfrequenz von f = 300 kHz abgetastet wird. Dann legt das Garn während einer Periode von T = 1/f = 3.3·10⁻⁶ s die Strecke s = v·T = 0.11 mm zurück, was wesentlich kleiner ist als die Durchmesser des Abtastbereiches 85, der ca. D = 1.5 mm beträgt. In diesem Beispiel ist also die Abtastfrequenz f genügend hoch gewählt. Für die Wahl der Abtastfrequenz f sollte vorzugsweise gelten: $f \geq 10 \cdot v/D$.

Die beiden LEDs 21, 22 sind in einem gemeinsamen Gehäuse 3 untergebracht. Das Gehäuse 3 ist voll vergossen aus einem für das Licht 71, 72 transparentem Kunststoff und hat eine gegen den Gampfad 91 gerichtete, gekrümmte Grenzfläche 31. Somit wirkt das Gehäuse 3 als Kollimatorlinse, welche das von den LEDs 21, 22 ausgesendete Licht 71, 72 möglichst zu parallelen Beleuchtungsstrahlenbündeln 73, 74 sammelt. Derartige verschiedenfarbige, in einem transparenten Gehäuse 3 vergossene Multi-LEDs 21, 22 sind kommerziell erhältlich, z. B. von den Firmen Nichia Corporation, Tokushima, Japan, oder Osram GmbH, München, Deutschland.

Es entspricht der Natur der Sache, dass sich die verschiedenen LEDs 21, 22 nicht am selben Ort befinden können, sondern an verschiedenen Orten innerhalb des Gehäuses 3 angebracht sind. Vorzugsweise liegen die beiden LEDs 21, 22 symmetrisch bezüglich einer optischen Achse 10 der Vorrichtung 1. Ihre gegenseitige Entfernung kann z. B. ca. 0.7 mm betragen. Wegen dieser Versetzung haben die beiden Beleuchtungsstrahlenbündel 73, 74 unterschiedliche Abstrahlungswinkel bezüglich der optischen Achse 10. Deshalb beleuchten die beiden LEDs 21, 22 verschiedene Beleuchtungsbereiche 81, 82 auf dem Gampfad 91, was schematisch in der Frontalansicht von **Figur 2** dargestellt ist. Ein roter Beleuchtungsbereich 81 liegt bezüglich der Gambewegungsrichtung 90 weiter stromaufwärts als ein grüner Beleuchtungsbereich 82, wobei die Versetzung der beiden Beleuchtungsbereiche 81, 82 ca. 1.5 mm und ihr Durchmesser jeweils ca. 3 mm betragen kann.

Die Vorrichtung 1 beinhaltet ferner einen Lichtdetektor 6 zur Detektion von Licht 75, das mit dem Garn 9 wechselwirkt. Im Ausführungsbeispiel von Figur 1 handelt es sich um vom Garn 9 bzw. am Garn 9 vorbei transmittiertes Licht 75. Eine detektorseitige Abbildungsoptik 4 bildet das Garn 9 auf dem Detektor 6 ab. Der Detektor 6 wandelt die auf ihn auftreffende Lichtintensität in ein zur Lichtintensität proportionales elektrisches Ausgangssignal um. Dieses Ausgangssignal wird auf einer elektrischen Leitung zu einer Auswerteeinheit geleitet, die beide nicht eingezeichnet sind. In der Auswerteeinheit wird das Ausgangssignal auf geeignete Weise demoduliert, um die einzelnen, den Lichtkomponenten 73, 74 entsprechenden Signalkomponenten zu erhalten. Die Signalkomponenten werden dann ausgewertet, vorzugsweise miteinander verknüpft, um eine Information über Parameter des Garns 9 wie Garndurchmesser und/oder im Garn 9 vorhandene Fremdstoffe zu erhalten. Die Auswertung kann analog und/oder digital erfolgen. Geeignete Verfahren und Einheiten zur Auswertung von Detektorsignalen beim Einsatz von verschiedenfarbigen Lichtquellen sind aus dem eingangs zitierten Stand der Technik an sich bekannt und brauchen hier nicht näher erläutert zu werden.

Zur klaren Beschreibung der Funktionsweise der Erfindung mit Hilfe der Mengenlehre wird nachfolgend die folgende Notation verwendet:

R = Menge aller Punkte im roten Beleuchtungsbereich 81, und
G = Menge aller Punkte im grünen Beleuchtungsbereich 82.

Würde sämtliches Licht von den beiden Beleuchtungsbereichen 81, 82 detektiert, d. h. sähe ein Detektor die Vereinigungsmenge $R \cup G$, so wären auch ausschliesslich rot beleuchtete Punkte aus einem roten Teilbereich 83, der Differenzmenge R\G, und ausschliesslich grün beleuchtete Punkte aus einem grünen Teilbereich 84, der Differenzmenge G\R, dabei. Die Erfassung von Messwerten aus solchen monochromatischen Teilbereichen 83, 84 könnte aber zu Fehlmessungen führen, weil die Auswertealgorithmen voraussetzen, dass gleichzeitig beide Lichtkomponenten 71, 72 detektiert werden. Hier setzt die vorliegende Erfindung an. Erfindungsgemäss wird ausschliesslich Licht aus der Schnittmenge R ∩ G der Beleuchtungsbereiche 81, 82, z. B. aus einem kreisscheibenförmigen Abtastbereich 85, detektiert. Die Menge A der Punkte aus dem Abtastbereich 85 ist also eine Teilmenge der Schnittmenge $R \cap G$ der Beleuchtungsbereiche 81, 82:

$$A \subseteq R \cap G \, ,$$

wobei A eine echte Teilmenge (A ⊂ R ∩ G) oder mit der Schnittmenge identisch (A = R ∩ G) sein kann. Zu diesem Zweck beinhaltet die Vorrichtung 1 eine Bildfeldblende 52, welche nur solches Licht 75 auf den Lichtdetektor 6 auftreffen lässt, das aus dem Abtastbereich 85 stammt.

Im Ausführungsbeispiel von Figur 1 ist die Bildfeldblende 52 als eigenes, unmittelbar vor oder auf dem Detektor 6 angeordnet optisches Element ausgeführt. Eine solche Bildfeldblende 52 kann jedoch auch durch die Grösse der lichtempfindlichen Fläche des Detektors 6 definiert sein, ohne dass zusätzliche optische Elemente benötigt werden.

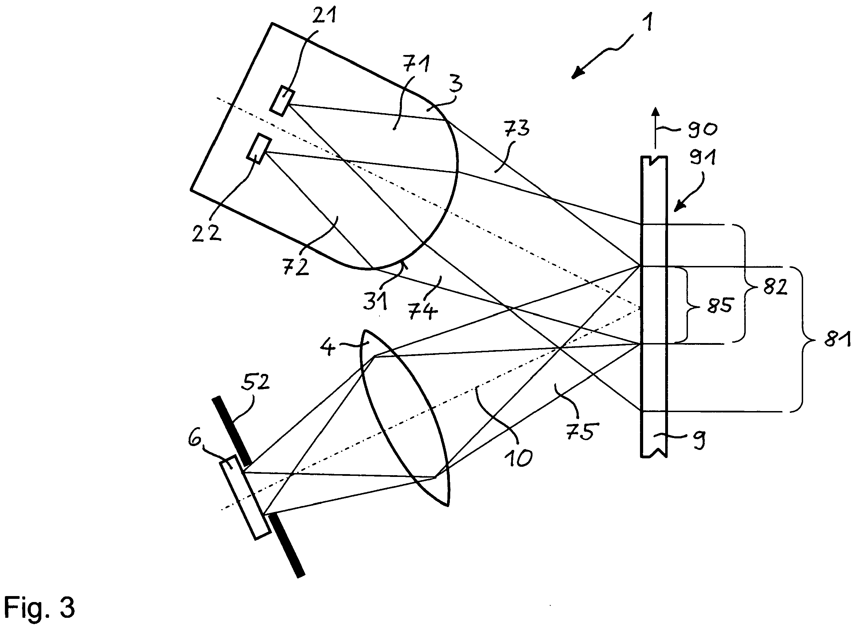

Eine zweite Ausführungsform der erfindungsgemässen Vorrichtung 1 ist in einer Seitenansicht in **Figur 3** dargestellt. Diese Ausführungsform ist analog zu derjenigen von Figur 1, so dass für einander entsprechende Elemente dieselben Bezugszeichen verwendet werden. Der wesentliche Unterschied zwischen den beiden Ausführungsformen besteht darin, dass in Figur 3 der Detektor 6 am Garn 9 reflektiertes Licht 75 detektiert. Die optische Achse 10 liegt hier nicht senkrecht, sondern schief zur Längsrichtung 90 des Garns 9. Dies ist aber zur Detektion von am Garn 9 reflektiertem Licht 75 nicht notwendig. Es ist möglich, die Lichtquellen 21, 22 (abgesehen vom gegenseitigen Versatz) und den Detektor 6 im Wesentlichen in einer Ebene anzuordnen, die senkrecht zur Längsrichtung 90 des Garns 9 steht. Falls in derselben Vorrichtung auch eine Transmissionsmessung gemäss Figur 1 erfolgen soll, kann der Detektor für die Transmissionsmessung ebenfalls in der genannten Ebene abgeordnet werden. Ein Garnreinigermesskopf mit Multi-LED-Lichtquellen, zwei Reflexionsdetektoren und einem Transmissionsdetektor, die im Wesentlichen in einer Ebene senkrecht zur Längsrichtung des Garns angeordnet sind, ist in den Schriften US-5,768,938 A oder WO-2004/044579 A1 offenbart. Eine solche Messkopfanordnung kann auch für die vorliegende Erfindung übernommen werden. Bei der in Figur 3 eingezeichneten Fresnelschen Lichtreflexion am Garn 9 handelt es sich um eine vereinfachte Idealisierung. Im Allgemeinen wird das Licht 73, 74 am Garn gestreut. Dies ändert aber nichts Wesentliches an der Wirkungsweise der vorliegenden Erfindung.

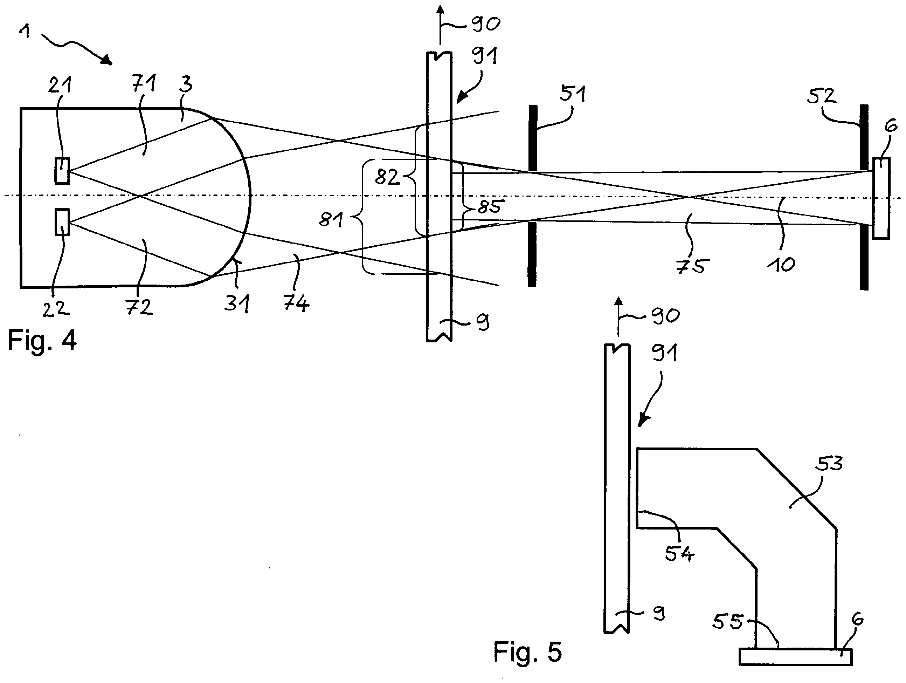

**Figur 4** zeigt eine dritte Ausführungsform der erfindungsgemässen Vorrichtung 1. Analog zur Ausführungsform von Figur 1 arbeitet diese Ausführungsform in Transmission, jedoch ohne Abbildungsoptik 4. Sie weist eine Gesichtsfeldblende 51 hinter dem Garn 9 und eine Bildfeldblende 52 vor dem Detektor 6 auf. Diese beiden Feldblenden 51, 52 bewirken bei geeigneter Gestaltung und Anordnung, dass nur Licht 75 aus dem Abtastbereich 85 auf den Detektor 6 gelangt.

Eine vierte Ausführungsform der detektorseitigen Lichtführung zeigt **Figur 5**. Hier wird das mit dem Garn 9 wechselwirkende Licht 75 in einem Lichtleiter 53 vom Pfad 91 zum Detektor 6 geführt. Der Lichtleiter 53 kann z. B. aus einem für das verwendete Licht transparenten Glas und/oder Kunststoff hergestellt sein. Seine Wände können verspiegelt sein, oder das geführte Licht kann mittels Totalreflexion an den Wänden reflektiert werden. Alternativ können die Wände streuend ausgestaltet sein, z. B. durch Aufrauhung und/oder eine Beschichtung. Erfindungsgemäss ist der Lichtleiter 53, d. h. seine Geometrie und seine optischen Eigenschaften, so ausgeführt und angeordnet, dass nur Licht 75 aus dem Abtastbereich 85 auf den Detektor 6 gelangt. Die Begrenzung einer garnseitigen Endfläche 54 wirkt hier als Gesichtsfeldblende 51 (siehe Figur 4), die Begrenzung einer detektorseitigen Endfläche 55 als Bildfeldblende 52. Ausserdem spielen hier auch die numerischen Aperturen der Endflächen 54, 55 eine Rolle bei der Festlegung des Abtastbereiches 85.

Auch zwischen den Lichtquellen 21, 22 können Lichtleiter vorhanden sein. Lichtleiter zur Lichtführung zwischen einer Lichtquelle bzw. Lichtdetektoren und dem Garn sind an sich in der US-5,768,938 A offenbart, ohne dass dort jedoch auf den Aspekt der Apertur- oder Feldblenden eingegangen wird.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Wie bereits erwähnt, kann die Vorrichtung 1 Lichtdetektoren sowohl für am Textilmaterial 9 transmittiertes als auch für am Textilmaterial 9 reflektiertes Licht aufweisen, d. h. als Kombination der Ausführungsformen der Figuren 1 und 3 ausgebildet sein. Für das transmittierte und/oder für das Reflektierte Licht können jeweils mehrere Detektoren vorhanden sein, vorzugsweise ein Detektor für das transmittierte und zwei Detektoren für das Reflektierte Licht.

In den Ausführungsformen der Figuren 1, 3 und 4 liegen beide Lichtquellen 21, 22 in einer Ebene, welche die Längsrichtung 90 des Garns 9 beinhaltet. Dies muss nicht notwendigerweise so sein. Es ist möglich, die Lichtquellen 21, 22 z. B. in einer Ebene senkrecht zur Längsrichtung 90 anzuordnen. Die Beleuchtungsbereiche 81, 82 von Figur 2 wären dann um 90° gedreht, der Abtastbereich 85 wäre aber im Wesentlichen derselbe. Eine derartige Vorrichtung wäre besonders robust gegenüber Garnlaufschwankungen. Der Fachmann versteht, dass weder die Beleuchtungsbereiche 81, 82 noch der Abtastbereich 85 kreisscheibenförmig zu sein brauchen.

Es können mehr als zwei Lichtquellen vorhanden sein, z. B. drei Lichtquellen mit den Farben Rot, Grün und Blau. Zusätzlich oder alternativ zu sichtbarem Licht kann das Textilmaterial mit ultravioletter und/oder infraroter Strahlung beleuchtet werden. Um die Lichtführung klarer darstellen und die Erfindung besser erläutern zu können, ist die Beleuchtung in den beiliegenden Figuren 1, 3 und 4 in Form von gerichteten Lichtstrahlenbündeln 73, 74 dargestellt. Es ist möglich und unter Umständen sogar vorteilhaft, stattdessen eine diffuse Beleuchtung des Pfades 91 vorzusehen. Auch das detektierte Licht 75 kann diffus sein, was z. B. durch eine streuende Ausgestaltung der Wände des Lichtleiters 53 (siehe Figur 5) oder durch eine Streuscheibe im empfängerseitigen Strahlengang erreicht werden kann.

BEZUGSZEICHENLISTE

1       Vorrichtung
10      optische Achse

21, 22  Lichtquellen

3       Gehäuse für die Lichtquellen
31      Grenzfläche des Gehäuses

4       Abbildungsoptik

51      Gesichtsfeldblende
52      Bildfeldblende
53      Lichtleiter
54      garnseitige Endfläche
55      detektorseitige Endfläche

6       Lichtdetektor

71, 72  ausgesendetes Licht
73, 74  Beleuchtungsstrahlenbündel
75      Licht aus dem Abtastbereich

81, 82  Beleuchtungsbereiche
83, 84  monochromatische Teilbereiche der Beleuchtungsbereiche
85      Abtastbereich

9       Textilmaterial
90      Längs- und Bewegungsrichtung des Textilmaterials
91      Pfad für das Textilmaterial

**Patentansprüche**

1. Vorrichtung (1) zur optischen Abtastung eines bewegten Textilmaterials (9), mit
einem Pfad (91) für das Textilmaterial (9),
mindestens zwei Lichtquellen (21, 22), die an verschiedenen Orten angeordnet sind und Licht (71, 72) mit verschiedenen spektralen Eigenschaften aussenden, zur Beleuchtung jeweils eines Beleuchtungsbereichs (81, 82) des Pfades (91),
einem Lichtdetektor (6) zur Detektion von aus den verschiedenen Beleuchtungsbereichen (81, 82) stammendem Licht und
mindestens einer Feldblende (51, 52), welche nur Licht (75) aus einem Abtastbereich (85), der eine Teilmenge der Schnittmenge aller Beleuchtungsbereiche (81, 82) ist, auf den Lichtdetektor (6) auftreffen lässt.

2. Vorrichtung (1) nach Anspruch 1, wobei die mindestens eine Feldblende (51, 52) durch eine Gesichtsfeldblende (51) und/oder eine Bildfeldblende (52) realisiert ist.

3. Vorrichtung (1) nach einem der vorangehenden Ansprüche, wobei die Lichtquellen (21, 22), der Pfad (91) und der Lichtdetektor (6) derart gegenseitig angeordnet sind, dass der Lichtdetektor (6) am Textilmaterial (9) transmittiertes oder vom Textilmaterial (9) reflektiertes Licht (75) detektiert.

4. Vorrichtung (1) nach Anspruch 3, wobei mehrere Lichtdetektoren vorhanden sind, von denen mindestens einer, vorzugsweise genau einer, am Textilmaterial (9) transmittiertes und mindestens ein anderer, vorzugsweise genau zwei, vom Textilmaterial (9) reflektiertes Licht detektieren.

5. Vorrichtung (1) nach einem der vorangehenden Ansprüche, wobei zur Lichtführung zwischen dem Pfad (91) und den Lichtquellen (21, 22) bzw. dem Lichtdetektor (6) Lichtübertragungselemente (53), bspw. Lichtleiter aus Glas und/oder Kunststoff, vorhanden sind.

6. Vorrichtung (1) nach einem der vorangehenden Ansprüche, wobei jede der Lichtquellen (21, 22) Licht (71, 72) aussendet, dessen Wellenlängenspektrum im Wesentlichen ein zusammenhängender, begrenzter Spektralbereich ist, und wobei sich die Spektralbereiche der verschiedenen Lichtquellen (21, 22) im Wesentlichen nicht überlappen.

7. Vorrichtung (1) nach Anspruch 6, wobei die Lichtquellen (21, 22) als Leuchtdioden ausgebildet sind.

8. Vorrichtung (1) nach Anspruch 7, wobei eine rotes Licht (71) aussendende Leuchtdiode (21) und eine grünes Licht (72) aussendende Leuchtdiode (22) vorhanden ist.

9. Vorrichtung (1) nach einem der vorangehenden Ansprüche, wobei die Lichtquellen (21, 22) in einem gemeinsamen Gehäuse (3), das vorzugsweise voll vergossen und für das ausgesendete Licht (71, 72) transparent ist, untergebracht sind.

10. Verwendung der Vorrichtung (1) nach einem der vorangehenden Ansprüche zur Detektion von Fremdstoffen im Textilmaterial (9), vorzugsweise in Garn.

11. Verfahren zur optischen Abtastung eines bewegten Textilmaterials (9), wobei das Textilmaterial (9) von mindestens zwei Lichtkomponenten (73, 74) mit verschiedenen spektralen Eigenschaften aus mindestens zwei Lichtquellen (21, 22), die an verschiedenen Orten angeordnet sind, in jeweils einem Beleuchtungsbereich (81, 82) beleuchtet wird,
aus den verschiedenen Beleuchtungsbereichen (81, 82) stammendes Licht detektiert wird und
nur Licht (75) aus einem Abtastbereich (85), der eine Teilmenge der Schnittmenge aller Beleuchtungsbereiche (81, 82) ist, detektiert wird.

12. Verfahren nach Anspruch 11, wobei am Textilmaterial (9) transmittiertes und/oder vom Textilmaterial (9) reflektiertes Licht (75) detektiert wird.

13. Verfahren nach Anspruch 11 oder 12, wobei das Wellenlängenspektrum einer jeden Lichtkomponente (73, 74) im Wesentlichen ein zusammenhängender, begrenzter Spektralbereich ist, und wobei sich die Spektralbereiche der verschiedenen Lichtkomponenten (73, 74) im Wesentlichen nicht überlappen.

14. Verfahren nach Anspruch 13, wobei das Textilmaterial (9) mit rotem Licht (73) und grünem Licht (74) beleuchtet wird.

15. Verfahren nach einem der Ansprüche 11-14, wobei das detektierte Licht (75) verwendet wird, um Fremdstoffe im Textilmaterial (9), das vorzugsweise ein Garn ist, zu detektieren.

## Claims

1. An apparatus (1) for optically scanning a moved textile material (9), comprising
a path (91) for the textile material (9),
at least two light sources (21, 22) which are arranged at different locations and which emit light (71, 72) with different spectral properties for illuminating one respective illumination area (81, 82) of the path (91),
a light detector (6) for detecting light originating from the various illumination areas (81, 82), and
at least one field stop (51, 52) which allows only light (75) from a scanning area (85) that is a subset of the intersection of all illumination areas (81, 82) to impinge on the light detector (6).

2. The apparatus (1) according to claim 1, wherein the at least one field stop (51, 52) is realized by an entrance pupil (51) and/or an exit pupil (52).

3. The apparatus (1) according to one of the preceding claims, wherein the light sources (21, 22), the path (91) and the light detector (6) are arranged with respect to each other in such a way that the light detector (6) will detect light (75) transmitted on the textile material (9) or reflected by the textile material (9).

4. The apparatus (1) according to claim 3, wherein several light detectors are provided, of which at least one, preferably precisely one, detects light transmitted on the textile material (9), and at least one other, preferably precisely two others, detects light reflected by the textile material (9).

5. The apparatus (1) according to one of the preceding claims, wherein light transmission elements (53), e.g. waveguides made of glass and/or plastic, are provided for light guidance between the path (91) and the light sources (21, 22).

6. The apparatus (1) according to one of the preceding claims, wherein each of the light sources (21, 22) emits light (71, 72), the wavelength spectrum of which is substantially a connected, delimited spectral range, and wherein the spectral ranges of the various light sources (21, 22) substantially do not overlap one another.

7. The apparatus (1) according to claim 6, wherein the light sources (21, 22) are arranged as light-emitting diodes.

8. The apparatus (1) according to claim 7, wherein where a light-emitting diode (21) emitting red light (71) and a light-emitting diode (22) emitting green light (72) are provided.

9. The apparatus (1) according to one of the preceding claims, wherein the light sources (21, 22) are housed in a common housing (3) which is preferably fully cast and is transparent for the emitted light (71, 72).

10. The use of the apparatus (1) according to one of the preceding claims for the detection of foreign matter in the textile material (9), preferably in yarn.

11. A method for optically scanning a moved textile material, wherein
the textile material (9) is illuminated by at least two light components (73, 74) with different spectral properties originating from at least two light sources (21, 22) which are arranged at different locations in one respective illumination area (81, 82),
light is detected which originates from the various illumination areas (81, 82), and only light (75) from a scanning area (85) which is a subset of the intersection of all illumination areas (81, 82) is detected.

12. The method according to claim 11, wherein light (75) that is transmitted on the textile material (9) and/or reflected by the textile material (9) is detected.

13. The method according to claim 11 or 12, wherein the wavelength spectrum of each respective light component (73, 74) is a substantially connected, delimited spectral range, and the spectral ranges of the various light components (73, 74) substantially do not overlap one another.

14. The method according to claim 13, wherein the textile material (9) is illuminated with red light (73) and green light (74).

15. The method according to one of the claims 11 to 14, wherein the detected light (75) is used in order to detect foreign matter in the textile material (9), which is preferably a yarn.

**Revendications**

1. Dispositif (1) de balayage optique d'une matière textile en mouvement (9), comportant
un chemin (91) pour la matière textile (9),
au moins deux sources de lumière (21, 22) disposées à des emplacements différents et émettant une lumière (71, 72) ayant des propriétés spectrales différentes pour éclairer une zone d'éclairage respective (81, 82) du chemin (91),
un détecteur de lumière (6) pour détecter la lumière provenant des différentes zones d'éclairage (81, 82) et
au moins un diaphragme de champ (51, 52) qui ne permet qu'à la lumière (75) provenant d'une zone de balayage (85), qui est un sous-ensemble de l'intersection de toutes les zones d'éclairage (81, 82), d'atteindre le détecteur de lumière (6).

2. Dispositif (1) selon la revendication 1, dans lequel ledit au moins un diaphragme de champ (51, 52) est réalisé par un diaphragme de champ de vue (51) et/ou un diaphragme de champ d'image (52).

3. Dispositif (1) selon l'une des revendications précédentes, dans lequel les sources de lumière (21, 22), le chemin (91) et le détecteur de lumière (6) sont mutuellement disposés de telle sorte que le détecteur de lumière (6) détecte la lumière (75) transmise sur la matière textile (9) ou réfléchie par la matière textile (9).

4. Dispositif (1) selon la revendication 3, dans lequel plusieurs détecteurs de lumière sont présents, dont au moins un, de préférence exactement un, détecte la lumière transmise sur la matière textile (9) et au moins un autre, de préférence exactement deux, détecte(nt) la lumière réfléchie par la matière textile (9).

5. Dispositif (1) selon l'une des revendications précédentes, dans lequel des éléments de transmission de lumière (53), par exemple des guides de lumière en verre et/ou en matière plastique, sont présents entre le chemin (91) et les sources de lumière (21, 22) ou le détecteur de lumière (6) pour guider la lumière.

6. Dispositif (1) selon l'une des revendications précédentes, dans lequel chacune des sources de lumière (21, 22) émet une lumière (71, 72) dont le spectre de longueurs d'onde est sensiblement une plage spectrale limitée continue et dans lequel les plages spectrales des différentes sources de lumière (21, 22) ne se chevauchent sensiblement pas.

7. Dispositif (1) selon la revendication 6, dans lequel les sources de lumière (21, 22) sont réalisées sous la forme de diodes électroluminescentes.

8. Dispositif (1) selon la revendication 7, dans lequel une diode électroluminescente (21) émettant une lumière rouge (71) et une diode électroluminescente (22) émettant une lumière verte (72) sont présentes.

9. Dispositif (1) selon l'une des revendications précédentes, dans lequel les sources de lumière (21, 22) sont logées dans un boîtier commun (3) qui est de préférence entièrement scellé et transparent pour la lumière émise (71, 72).

10. Utilisation du dispositif (1) selon l'une des revendications précédentes pour la détection de substances étrangères dans la matière textile (9), de préférence dans un fil.

11. Procédé de balayage optique d'une matière textile en mouvement (9), dans lequel
la matière textile (9) est éclairée par au moins deux composantes lumineuses (73, 74) ayant des propriétés spectrales différentes, provenant d'au moins deux sources de lumière (21, 22) qui sont disposées à des emplacements différents, dans des zones d'éclairage respectives (81, 82),
la lumière provenant des différentes zones d'éclairage (81, 82) est détectée et
seule la lumière (75) provenant d'une zone de balayage (85), qui est un sous-ensemble de l'intersection de toutes les zones d'éclairage (81, 82), est détectée.

12. Procédé selon la revendication 11, dans lequel la lumière (75) transmise sur la matière textile (9) et/ou réfléchie par la matière textile (9) est détectée.

13. Procédé selon la revendication 11 ou 12, dans lequel le spectre de longueurs d'onde de chaque composante lumineuse (73, 74) est sensiblement une plage spectrale limitée continue et dans lequel les plages spectrales des différentes composantes lumineuses (73, 74) ne se chevauchent sensiblement pas.

14. Procédé selon la revendication 13, dans lequel la matière textile (9) est éclairée par une lumière rouge (73) et une lumière verte (74).

15. Procédé selon l'une des revendications 11 à 14, dans lequel la lumière détectée (75) est utilisée pour détecter des substances étrangères dans la matière textile (9), qui est de préférence un fil.

Fig. 1

Fig. 2

Fig. 3

Fig. 4

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9313407 A1
- US 5383017 A
- US 5414520 A
- US 5768938 A
- EP 1655599 A2
- EP 1655600 A2
- WO 0020849 A1
- EP 0399945 A2
- EP 0652432 A1
- WO 0073771 A1
- WO 2007010325 A1
- WO 03008950 A2
- EP 1018645 A1
- WO 9529396 A1
- WO 2004044579 A1
- WO 2007012936 A2

### In der Beschreibung aufgeführte Nicht-Patentliteratur

- **E. HECHT ; A. ZAJAC.** Optics. Addison-Wesley Publishing Company, 1974